# EUROPÄISCHE PATENTANMELDUNG

(11) **EP 1 731 095 A2**
(43) Veröffentlichungstag der Anmeldung: **13.12.2006**
(21) Anmeldenummer: 06090097.4
(22) Anmeldetag: 02.06.2006
(51) Int. Cl.: A61B 5/08

(54) **Messung und Analyse von Bestandteilen von exhaliertem Atemgas**

(30) Priorität: 06.06.2005 DE 102005026933
(71) Anmelder: FILT Lungen- und Thoraxdiagnostik GmbH, D-13125 Berlin (DE)
(72) Erfinder: Becher, Gunther, 16321 Schönow (DE)
(74) Vertreter: Kietzmann, Manfred

(57) **Zusammenfassung**

Die Erfindung betrifft ein Verfahren zur Messung und Analyse von Bestandteilen des exhaliertem Atemgases bei dem eine zeitproportionale Messung des oder der Marker in der Ausatemluft und von sich verändernden Atemgrößen der exhalierten Atemluftmenge erfolgt, die tendenziell nichtlinear und umgekehrt proportional zueinander verlaufenden Messkurven rechentechnisch mit artifiziellen Messkurven aus Messungen bei konstantem Flow und/oder gegen eine Stenose verglichen und die Ist-Messwerte des oder der Marker bezogen auf einen konstanten Wert der jeweiligen Atemgröße extrapoliert und ausgegeben werden oder als Messwert die Ausscheidung des Markers pro Zeiteinheit und/oder pro Einheit Ausatemluft berechnet und ausgegeben wird (Fig. 1).

## Beschreibung

Die Erfindung betrifft die Messung und Analyse von Atemgas, insbesondere von exhalierten Markern, die nicht mit dem eigentlichen Gaswechsel in Beziehung stehen.

Marker wie Wasserstoffperoxid, NO (Stickstoffmonoxid), Ammonium, Nitrat, Nitrit, Acrylate, Alkene, Aldehyde (z. B. Malondialdehyd) werden aus Zellen der Atemwege (Monozyten, Leukozyten, Mastzellen, Eosinophile) kontinuierlich ohne Beziehung zum Atemgasstrom freigesetzt. Desgleichen geschieht aus den Bronchialepithel und dem Alveolarepithel. Diese Marker können als gasförmige und auch als nichtgasförmige Marker im Atemgasstrom und im Kondensat der Ausatemluft gefunden werden.

Verfahren und Vorrichtungen zu deren Messung und zur Analyse von Atemgas sind bekannt.

Durch die WO 91/05255 A1 ist ein Verfahren zur Diagnose des Gesundheitszustandes sowie zur Therapieüberwachung und zur Verlaufskontrolle von Krankheiten, speziell der Lunge und der Atemwege, bekannt, bei dem nicht-flüchtige Substanzen - gegebenenfalls zusammen mit endogenen und exogen flüchtigen Stoffen - in der menschlichen Atemluft analysiert werden. Hierzu werden von einem Probanden Atemstöße auf ein auf -196°C gekühltes, 1cm² großes Probenträgerplättchen aufgehaucht. Die Probe wird dann auf dem Cryotisch durch Anlagen eines Ultrahochvakuums gefriergetrocknet und anschließend in unterschiedlicher Weise analysiert.

Aus der EP 0 759 169 B1 ist ein Verfahren zum Sammeln von ausgeatmetem Atemkondensat für die Diagnose des Gesundheitszustandes und von Stoffwechselleistungen der Lunge und der Atemwege sowie anderer Organe und zur Analyse ausgeatmeter körperfremder Stoffe, bekannt, bei dem die Ausatemluft ein Probensammelrohr durchströmt und in diesem auf eine Minustemperatur unter 0°C abgekühlt wird, wobei die flüssigen und lösbaren Bestandteile auskondensieren und an der, eine niedrige Temperatur als das Atemkondensat aufweisenden, Innenwand des Probensammelrohres anfrieren.

Die DE 199 51 204 C2 beschreibt ein Verfahren zur Analyse der Inhaltstoffe der Ausatemluft, bei dem aus der Ausatemluft gewonnenes Atemkondensat einer Analyse unterzogen und das Ergebnis angezeigt wird, wobei die Menge des aus der Ausatemluft gewonnenen Atemkondensates gemessen wird und nach dem Erreichen einer vorgegebenen Probenmenge eine direkte Bestimmung der enthaltenen Substanzen durch Messung von Einzel- und/oder Summenparametern mittels eines oder mehreren elektrochemischen Sensoren erfolgt.
Der oder die Sensoren sind dem Filter oder der Speicherschicht so nachgeordnet, dass durch den Filter oder die Speicherschicht diffundiertes Atemkondensat unmittelbar auf den oder die Sensoren gelangt.

Ein weiteres Verfahren zur Bestimmung von Parametern eines Atemkondensats unter Verwendung eines oder mehrer Sensoren zum Messen der Parameter des Atemkondensats und einer Auswerteeinheit mit Anzeige für die Messergebnisse, wird in der DE 101 37 565 B4 beschrieben.

Mit diesem Verfahren wird in kompakter Weise möglichst vielen Randbedingungen entsprochen, indem innerhalb einer geschlossenen Kassette, in der sich der oder die Sensoren befinden, aus Vorratsbehältern der Kassette durch eine Einwirkung von außerhalb der Kassette auf die Vorratsbehälter Lösungen ausgebracht und auf den oder die Sensoren aufgebracht werden, wobei die Lösungen eine Kassettenspülung vornehmen und/oder eine Konditionierung des oder der Sensoren bewirken und/oder eine Kalibrierung des oder der Sensoren vornehmen und/oder zur Verdünnung der Probenlösung und/oder zur Erhöhung deren Ionenkonzentration oder Leitfähigkeit beitragen.
Durch eine Öffnung der Kassette wird eine Probenlösung aus oder mit Atemkondensat auf den oder die Sensoren zur Parameterbestimmung aufgebracht, wobei die Temperatur des oder der Sensoren und die der Probenlösung während der Bestimmung der Parameter des Atemkondensats aufeinander abgestimmt geregelt werden.
Neben den reinen Messergebnissen werden auch Ablaufdaten innerhalb der Kassette wie Durchfluss, Temperatur, Luftblasenfreiheit, Zeit erfasst und der Auswerteeinheit zugeleitet.

Das Verfahren zur Quantifizierung der Austauschprozesse in den Atemwegen unter Verwendung von Atemkondensat gemäß DE 101 37 566 A1 sieht vor, dass körperfremde Stoffe in Form von Markersubstanzen oral oder durch intravenöse oder subkutane Injektion verabreicht werden, oder körpereigene isotopenmarkierte Markersubstanzen in Form von Peptiden und Proteinen sowie deren metabolischen Vorstufen und Abbauprodukte, isotopenmarkierten Zucker und Polysacchariden sowie deren metabolischen Vorstufen und Abbauprodukte oder von isotopenmarkierten Lipiden, sowie deren metabolische Vorstufen und Abbauprodukte durch intravenöse oder subkutan Injektion verabreicht werden und nach Einstellung des Verteilungsgleichgewichtes im Körper Atemkondensat gewonnen wird und die Markersubstanz im Atemkondensat quantitativ bestimmt wird.

Bei diesen und weiteren Verfahren wird als bekannt vorausgesetzt, dass die Konzentration in der Ausatemluft und/oder in deren Kondensat abhängig ist vom Ausatemgasstrom (Strömungsgeschwindigkeit). Der Patient ist so gehalten, durch seine Atemtechnik eine optimale Konzentration zu gewährleisten.

Die gegenwärtigen Guidelines der ATS und ERS empfehlen daher eine konstante Ausatmung gegen einen Ausatemwiderstand, um die Marker zu messen, zuletzt das erst am 14. April 2005 veröffentlichte Guidelines für NO-Messung. (American Thoracic Society Documents: ATS/ERS Recommendations for Standardized Procedures for the Online and Offline Measurement of Exhaled Lower Respiratory Nitric Oxide and NasalNitric Oxide, 2005. Am J Respir Crit Care Med Vol 171. pp 912-930, 2005)

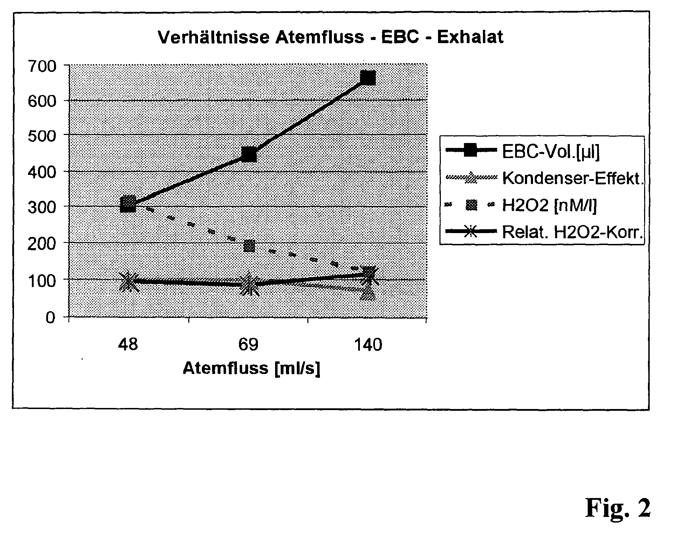

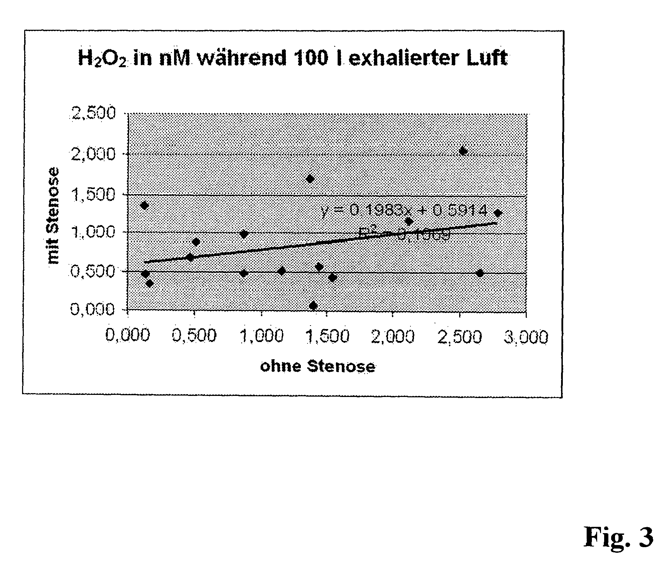

Die in den Fig. 2-4 dargestellten Diagramme zeigen diesen Zusammenhang am Beispiel.

Im einzelnen werden dargestellt:
- Fig. 2:: Verhältnis Atemfluss-EBC-Extrakt (Schleiss et al, Eur Respir J 2000; 16: 1115-1118)
- Fig. 3:: H₂O₂ in nM während 100 l exhalierter Luft mit und ohne Stenose (Fraktionierte Atemkondensatsammlung zur Gewinnung spezifischer Marker G. Becher. 46. Kongress der Deutschen Gesellschaft für Pneumologie, vom 16.-19. März 2005 in Berlin)

Auch bei Atmung gegen eine exspiratorische Stenose zeigt sich eine klare Beziehung der Exhalation von H₂O₂ über die Zeit besser als zur Konzentration im Kondensat.

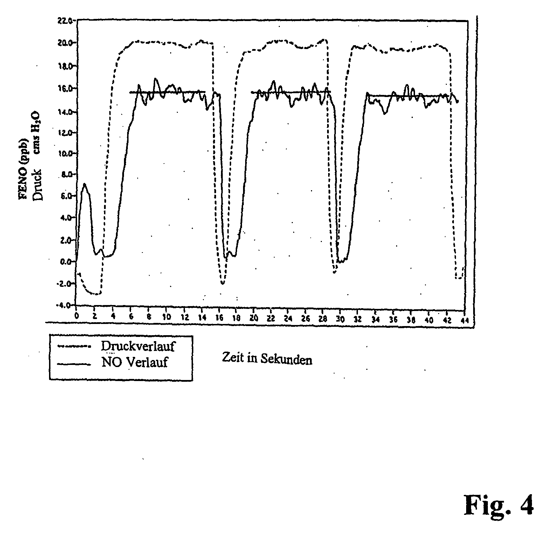

Das in Fig. 4 dargestellte Diagramm zeigt das vorgeschriebene Druckprofil der Ausatmung, um ein Plateau in der NO Konzentration zu erreichen. (American Thoracic Society Documents: ATS/ERS Recommendations for Standardized Procedures for the Online and Offline Measurement of Exhaled Lower Respiratory Nitric Oxide and NasalNitric Oxide, 2005. Am J Respir Crit Care Med Vol 171. pp 912-930, 2005)

Die bisherige Analyseempfehlungen bzw. Messmethoden fordern deshalb zurecht einen gleichmäßigen Luftfluss (z. B. bei NO von 50 ml/sec +/- 10%). Das ist speziell für Patienten mit Atemeinschränkungen nicht leicht. Sie müssen die damit verbundenen Atemmanöver realisieren.

Aufgabe der Erfindung ist es, bei der Messung und Analyse von Atemgas, insbesondere von Markern, die nicht mit dem eigentlichen Gaswechsel in Beziehung stehen, ohne die für einen gleichmäßigen Luftfluss notwendigen Atemmanöver auszukommen.

Gelöst wird diese Aufgabe mit den Merkmalen des Anspruches 1 und 12, vorteilhafte Ausgestaltungen sind Gegenstand der Unteransprüche.

Das Verfahren geht dabei davon aus, dass die sinkende Konzentration von exhalierten Markern (z. B. NO, CO, H₂O₂, Ammonium) ursächlich darauf zurückzuführen ist, dass das von der Schleimhaut gleichmäßig freigesetzte Material (entsprechend Stoffumsatz der Zellen der Schleimhaut und der Gesamtfläche) durch unterschiedliche vorbeistreifende Mengen Ausatemluft aufgenommen wird.

Das erfindungsgemäße Verfahren zur Messung und Analyse von exhaliertem Atemgas sieht deshalb vor, dass eine zeitproportionale Messung des oder der Marker in der Ausatemluft und von sich verändernden Atemgrößen der exhalierten Atemluftmenge erfolgt, die tendenziell nichtlinear und umgekehrt proportional zueinander verlaufenden Messkurven rechentechnisch mit artifiziellen Messkurven aus Messungen bei konstantem Flow und/oder gegen eine Stenose verglichen und die Ist-Messwerte des oder der Marker bezogen auf einen konstanten Wert der jeweiligen Atemgröße extrapoliert und ausgegeben werden oder als Messwert die Ausscheidung des Markers pro Zeiteinheit und/oder pro Einheit Ausatemluft berechnet und ausgegeben wird.

Als sich verändernde Atemgrößen der exhalierten Luft können gemessen werden: Atemfluss und/oder Atemvolumen und/oder BTPS-Bedingungen.

In Abhängigkeit von der Ansprechgeschwindigkeit des oder der Sensoren für den oder die Marker ist eine atemsynchrone Auswertung (Anzeige) vorgesehen oder eine kumulierte Messung des Markers über eine bestimmte Zeit.
Eine kumulierte Messung erfolgt vorteilhafterweise durch Messung der Konzentration im Mischgas im Ausatembeutel (Douglas Sack), ansonsten kann es auch durch Sammlung des Kondensates der Ausatemluft erfolgen. Die Auswertung erfolgt dann gemäss Sammeldauer und während der Sammlung ausgeatmeter Luft.

Um insbesondere den rechentechnischen Aufwand zu senken und die Beziehung zwischen den beiden Messkurven (Marker und Atemfluss) möglichst einfach herzustellen, wird empfohlen, dass die zeitproportionalen Messungen bei Ruheatmungsbedingungen erfolgen. Solche Einflussfaktoren wie zugrundeliegende Fläche der Atemwege und der Lunge, Füllzustand, Durchblutung, Verteilungsstörungen sind auf diese Weise besser beherrschbar. Der besondere Vorteil der Methode besteht darin, dass der Proband (die Versuchsperson etc) keine vorgebenen Atemmanöver durchführen muss sondern beliebig atmen kann. Die Methode wird dadurch "mitarbeitsunabhängig" .und somit auch für schwerkranke Patienten mit Atemstörungen und Kinder nutzbar, selbst für Tiere.

Dies betrifft insbesondere die rechentechnische Korrelation der gesuchten Marker mit:
- Lungenvolumen und/oder
- Atemvolumen und/oder
- Atemstrom und/oder
- Atemgasen wie Sauerstoffaufnahme oder Kohlendioxidabgabe und/oder mit
- verschiedenen simultan gemessenen Markern zur Erkennung ihrer internen Beziehung oder Abhängigkeit voneinander.

Weiterhin ist vorgesehen, dass die Messung der Marker direkt in der Gasphase und/oder im Atemkondensat erfolgt.

Das Kondensat wird dabei auf dem direkt im Atemstrom befindlichen Sensor und/oder auf einer darauf befindlichen Speicher- oder Abscheideschicht gebildet.

Der oder die Speicher- und/oder Abscheideschichten für Konzentrat können dafür derart präpariert sein, dass sie eine Wasserdampf- bzw. Dampfabscheidung allgemein begünstigen.

Bei einer bevorzugten Ausgestaltung sind die Speicher- und/oder Abscheideschicht(-en) mit einer die leitfähig, des Kondensats begünstigenden Vorlage, vorzugsweise einer aus Salz versehen. Das gebildete Kondensat bekommt so eine gewisse elektrische Leitfähigkeit, die die Messung mit einem amperometrischen Sensor begünstigt.

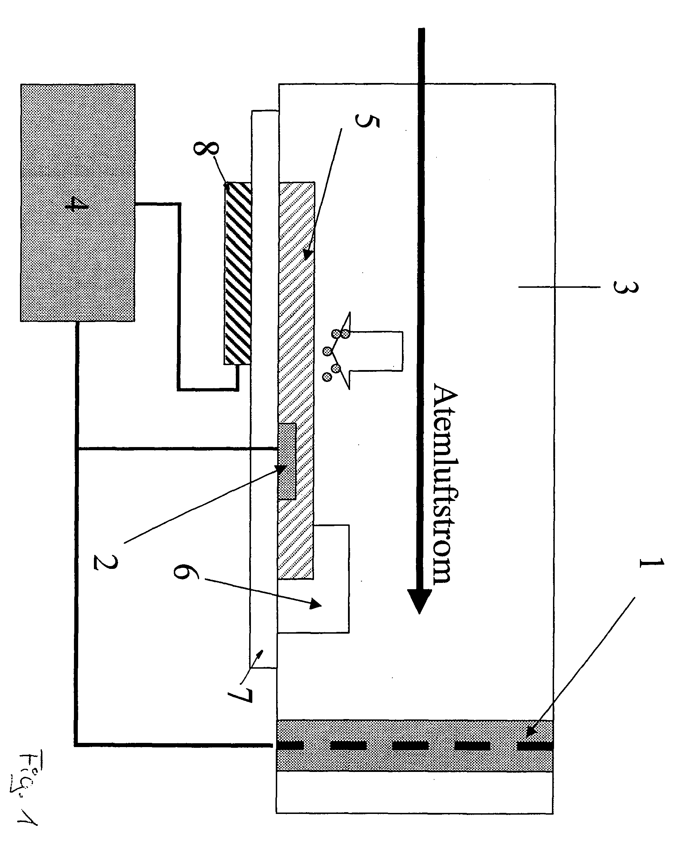

Eine Vorrichtung zur Durchführung des Verfahrens, bei dem eine zeitproportionale Messung des oder der Marker in der Ausatemluft und von sich verändernden Atemgrößen der exhalierten Atemluftmenge erfolgt, ist als Prinzipskizze in Fig. 1 dargestellt.

Mindestens ein Messgerät 1 oder ein Messsensor zur Bestimmung von Atemgrößen und ein Messgerät 2 oder ein Messsensor zur Bestimmung eines oder mehrerer Marker sind an oder in einer vom Atemstrom durchflossenen Leitung 3 angeordnet.

Die Messgeräte 1, 2 oder Sensoren oder ein Sensor und ein Messgerät können baulich zu einer Messeinheit vereint sein.

Die ermittelten Messwerte oder daraus gewonnene Daten werden in mindestens einem Rechner 4 zusammengeführt, der die tendenziell nichtlinear und umgekehrt proportional zueinander verlaufenden Messkurven (Marker, Atemgrösse) mit artifiziellen Messkurven aus Messungen bei konstantem Flow und/oder gegen eine Stenose vergleicht und die Ist-Messwerte des oder der Marker bezogen auf einen konstanten Wert der jeweiligen Atemgröße extrapoliert und ausgibt oder als Messwert die Ausscheidung des Markers pro Zeiteinheit und/oder pro Einheit Ausatemluft berechnet und ebenfalls ausgibt.

Die Messgeräte- oder Sensoranordnung an oder in der Leitung 3 kann durch Kombination eines geeigneten Atemstrommessgerätes (Venturi-Rohr, Fleisch'sche Düse, Flügelradspiometer, Thermistor, Ultraschall-Spirometer, Staudruckmessung) mit einem Sensor für den oder die gesuchten Marker, wie
- Biosensor für H₂O₂-Bestimmung,
- Radikalsensor für Superoxidbestimmung,
- Mehrfach-Biosensor auf Dickschichtbasis,
- CO₂-Sensor,
- Sauerstoffsensor,
- pH-Mess-Sonde,
- Lactatsensor,
- Ammoniumsensor und/oder
- Nitrat-Nitrit-Sensor
- Antikörperbeschichtete Biochips, (die das spezielle Antigen in einer immunologischen Reaktion binden, wobei nichtgebundene unspezifische Ablagerungen bzw. Ausfällungen auf dem Chip vor der Analyse (z.B. Farbreaktion) abgewaschen werden).
gebildet sein.

Wird entsprechend der Ansprechgeschwindigkeit des oder der Sensoren für den oder die Marker anstelle einer atemsynchrone Auswertung eine kumulierte Messung des Markers über eine bestimmte Zeit vorgesehen, wird die Leitung 3 durch einen Ausatembeutel, vorzugsweise ein Douglas-Sack ersetzt.

Weiterhin ist vorrichtungsseitig vorgesehen, dass mindestens ein Sensor, der im Atemkondensat in der Leitung 3 angeordnet ist, über eine Speicher- und/oder Abscheideschicht(-en) 5 für Kondensat verfügt. Dabei kann die Speicher- oder Abscheideschicht 5 über Partikel oder über eine solche Beschichtung oder eine solche Schicht verfügen, die die Wasserdampf- oder die Dampfabscheidung begünstigt.

Bewährt haben sich hygroskopische Beschichtungen oder eine Kühlschicht z.B. in Form eines untergebauten Peltierelementes 8.

Mit dem vorgeschlagenen Verfahren und der entsprechenden Vorrichtung werden die bisherige Analyseempfehlungen bzw. Messmethoden, die einen gleichmäßigen Luftfluss fordern, hinfällig. Es wird der Weg zu einer neuen Generation von Messgeräten zur Messung und Analyse von exhaliertem Atemgas eröffnet.

### Bezugszeichenliste

- 1: Messgerät/-sensor für Atemgrößen (Atemfluss)
- 2: Messgerät/-sensor für Marker
- 3: Leitung (Atemrohr)
- 4: Rechner (Auswerteeinheit)
- 5: Speicherschicht
- 6: Reservoir
- 7: Träger
- 8: Peltierelement

## Patentansprüche

1. Verfahren zur Messung und Analyse von Bestandteilen des exhaliertem Atemgases
bei dem eine zeitproportionale Messung des oder der Marker in der Ausatemluft und von sich verändernden Atemgrößen der exhalierten Atemluftmenge erfolgt,
die tendenziell nichtlinear und umgekehrt proportional zueinander verlaufenden Messkurven rechentechnisch mit artifiziellen Messkurven aus Messungen bei konstantem Flow und/oder gegen eine Stenose verglichen und die Ist-Messwerte des oder der Marker bezogen auf einen konstanten Wert der jeweiligen Atemgröße extrapoliert und ausgegeben werden
oder als Messwert die Ausscheidung des Markers pro Zeiteinheit und/oder pro Einheit Ausatemluft berechnet und ausgegeben wird.

2. Verfahren nach Anspruch 1, **dadurch gekennzeichnet, dass**
als sich verändernde Atemgrößen der exhalierten Luft gemessen werden: Atemfluss und/oder Atemvolumen und/oder BTPS-Bedingungen.

3. Verfahren nach Anspruch 1 oder 2, **dadurch gekennzeichnet, dass**
die zeitproportionalen Messungen bei Ruheatmungsbedingungen erfolgen.

4. Verfahren nach einem der Ansprüche 1 bis 3, **dadurch gekennzeichnet, dass**
rechentechnisch eine Korrelation der gesuchten Marker mit:
- Lungenvolumen und/oder
- Atemvolumen und/oder
- Atemstrom und/oder
- Atemgasen wie Sauerstoffaufnahme oder Kohlendioxidabgabe und/oder mit
- verschiedenen simultan gemessenen Markern zur Erkennung ihrer internen Beziehung oder Abhängigkeit voneinander
hergestellt wird.

5. Verfahren nach einem der Ansprüche 1 bis 4, **dadurch gekennzeichnet, dass**
entsprechend der Ansprechgeschwindigkeit des oder der Sensoren für den oder die Marker eine atemsynchrone Auswertung (Anzeige) erfolgt oder eine kumulierte Messung des Markers über eine bestimmte Zeit.

6. Verfahren nach Anspruch 5, **dadurch gekennzeichnet, dass**
eine kumulierte Messung durch Messung der Konzentration im Mischgas im Ausatembeutel (Douglas Sack) erfolgt.

7. Verfahren nach einem der Ansprüche 1 bis 6, **dadurch gekennzeichnet, dass**
die Messungen der Marker direkt in der Gasphase und/oder im Atemkondensat erfolgen.

8. Verfahren nach Anspruch 7, **dadurch gekennzeichnet, dass**
das Kondensat auf dem direkt im Atemstrom befindlichen Sensor und/oder auf einer darauf befindlichen Speicher- oder Abscheideschicht(-en) gebildet wird.

9. Verfahren nach Anspruch 8, **dadurch gekennzeichnet, dass**
mindestens eine Speicher- und/oder Abscheideschicht für Konzentrat derart präpariert ist, dass sie eine Wasserdampf bzw. Dampfabscheidung allgemein begünstigt.

10. Verfahren nach Anspruch 9, **dadurch gekennzeichnet, dass**
der Speicher- und/oder die Abscheideschicht mit einer die Leitfähigkeit des Kondensats begünstigenden Vorlage, vorzugsweise einer aus Salz (bzw. Elektrolytmischungen) versehen wird.

11. Verfahren nach Anspruch 10, **dadurch gekennzeichnet, dass**
die Messung des oder der Marker mit amperometrischen Sensoren erfolgt.

12. Vorrichtung zur Durchführung des Verfahrens nach Anspruch 1, bei dem eine zeitproportionale Messung des oder der Marker in der Ausatemluft und von sich verändernden Atemgrößen der exhalierten Atemluftmenge erfolgt, umfassend
mindestens ein Messgeräte (1) oder ein Messsensor zur Bestimmung von Atemgrößen und ein Messgerät (2) oder ein Messsensor zur Bestimmung eines oder mehrerer Marker an oder in einer vom Atemstrom durchflossenen Leitung (3) angeordnet, wobei die ermittelten Messwerte oder daraus gewonnene Daten mindestens in einem Rechner (4) zusammengeführt sind.

13. Vorrichtung nach Anspruch 12, **dadurch gekennzeichnet, dass**
die Messgeräte (1, 2) oder Sensoren oder ein Sensor und ein Messgerät baulich zu einer Messeinheit vereint sind.

14. Vorrichtung nach Anspruch 12 oder 13, **dadurch gekennzeichnet, dass**
die Messgeräte- oder Sensoranordnung an der Leitung (3) oder die Messeinheit mit simultaner Bestimmung von Atemstrom und Markern, insbesondere biochemischen, durch Kombination eines geeigneten Atemstrommessgerätes (Venturi-rohr, Fleisch'sche Düse, Flügelradspiometer, Thermistor, Ultraschall-Spirometer, Staudruckmessung) mit einem Sensor für den oder die gesuchten Marken, wie
- Biosensor für H₂O₂-Bestimmung,
- Radialsensor für Superoxidbestimmung,
- Mehrfach-Biosensor auf Dickschichtbasis,
- CO₂-Sensor,
- Sauerstoffsensor,
- pH-Mess-Sonde,
- Lactatsensor,
- Ammoniumsensor und/oder
- Nitrat-Nitrit-Sensor und/oder
- Immunologischer Sensor (Antikörperbeschichteter Biochip) gebildet ist.

15. Vorrichtung nach einem der Ansprüche 12 bis 14, **dadurch gekennzeichnet, dass**
die Messgeräte (1) und (2) bzw. entsprechende Sensoren eine zeitlich gleiche oder angenäherte Ansprechdauer aufweisen.

16. Vorrichtung nach Anspruch 12, **dadurch gekennzeichnet, dass**
die Leitung (3) ein Ausatembeutel, vorzugsweise ein Douglas-Sack ist.

17. Vorrichtung nach einem der Ansprüche 12 bis 14, **dadurch gekennzeichnet, dass**
mindestens ein Sensor, der im Atemstrom in der Leitung (3) angeordnet ist, über eine Speicher- und/oder Abscheideschicht (5) für Kondensat verfügt.

18. Vorrichtung nach Anspruch 17, **dadurch gekennzeichnet, dass**
die Speicher- oder Abscheideschicht(-en) (5) über Partikel oder eine solche Beschichtung oder eine Schicht verfügt, die die Wasserdampf- oder die Dampfabscheidung begünstigt.

19. Vorrichtung nach Anspruch 17 oder 18, **dadurch gekennzeichnet, dass**
die Speicher- oder Abscheideschicht(-en) (5) über eine Salzvorlage verfügt.

20. Vorrichtung nach Anspruch 19, **dadurch gekennzeichnet, dass**
die Messung des oder der Marker mittels eines oder mehrerer amperometrischen Sensoren erfolgt.
